## Europäisches Patentamt
## European Patent Office
## Office européen des brevets

(11) Publication number: **0 164 888**
**B1**

# EUROPEAN PATENT SPECIFICATION

(45) Date of publication of patent specification: **15.03.89**

(21) Application number: **85303236.5**

(22) Date of filing: **07.05.85**

(51) Int. Cl.⁴: **C 12 M 3/02,** C 12 M 1/12, C 12 M 1/10

(54) Cell and tissue culture process and apparatus.

(30) Priority: **04.05.84 JP 88345/84**

(43) Date of publication of application:
**18.12.85 Bulletin 85/51**

(45) Publication of the grant of the patent:
**15.03.89 Bulletin 89/11**

(84) Designated Contracting States:
**DE FR GB**

(56) References cited:
BE-A- 672 987
DE-A-2 934 328
GB-A-2 097 817
US-A-3 970 518
US-A-4 223 094

BIOTECHNOLOGY AND BIOENGINEERING, vol. 25, no. 10, October 1983, pages 2359-2370, John Wiley & Sons, Inc., New York, US; HIDEO TANAKA et al.: "Rotating drum fermentor for plant cell suspension cultures"

The file contains technical information submitted after the application was filed and not included in this specification

(73) Proprietor: **JAPAN SYNTHETIC RUBBER CO., LTD.**
**11-24, Tsukiji-2-chome Chuo-ku**
**Tokyo 104 (JP)**

(72) Inventor: **Tosaki, Chikao**
**26-12, Higashiyurigaoka-4-chome**
**Asao-ku Kawasaki-shi (JP)**

Inventor: **Yamada, Keiichi**
**JSR Aobaryo 29, Aobadai-2-chome**
**Midori-ku Yokohama (JP)**

Inventor: **Kariya, Masao**
**2-202, JSR Shataku 29, Aobadai-2-chome**
**Midori-ku Yokohama (JP)**

(74) Representative: **Tubby, David George et al**
**MARKS & CLERK 57-60 Lincoln's Inn Fields**
**London WC2A 3LS (GB)**

Courier Press, Leamington Spa, England.

**Description**

The present invention relates to a process and apparatus for culturing cells or plant tissues, and is particularly suitable for the production of physiologically active substances.

Physiologically active substances are substances which play some role in the reactions or activity of cells or plant tissues. Some such substances can be synthesized, but commonly they are produced by living cells or plant tissues, either in the body of the organism from which the cells or tissues are derived or by culture of independent cells or tissues.

In cell culture, the cells can either be anchorage-independent (which means that they can propagate whilst suspended in a suitable liquid medium containing the necessary nutrients for the cells) or anchorage-dependent (which means that the cells must first be adhered to the surface of a substrate before they will propagate). Recently, relatively small diameter particles have been used as the substrate for anchorage-dependent cells, because such carrier particles provide a large area to which the cells may adhere. The anchorage-independent cells or anchorage-dependent cells on a substrate are suspended in a liquid medium containing nutrients for the cells so that the cells are in contact with the liquid medium.

In order that the cell culture should take place efficiently, it is important that the cells to be cultured should always be in contact with fresh medium. In the case of anchorage-independent cells, this necessitates that the cells should be highly dispersed in the liquid medium. Similarly, in the case of anchorage-dependent cells adhering to carrier particles, the carrier particles should be highly dispersed in the liquid medium, so that the surfaces of these particles can be used effectively and evenly; this allows a high proportion of the particles to be used for supporting the cells, which also enhances culture efficiency.

The conventional method of ensuring that cells (whether anchorage-independent cells or anchorage-dependent cells suspended on carrier particles, and including plant tissues) are in contact with fresh liquid medium is to stir the system comprising the liquid medium and the cells with rotating blades. This applies a shear force to the system and inevitably damages the cells during culture as a result of collision of the cells with the rotating blades or by the action of the strong shear forces; this reduces culture efficiency and, with certain types of fragile cells, prohibits the use of this method. When the method is carried out on a large scale for mass culture, a larger stirrer is required and inevitably the shear force becomes greater, further reducing culture efficiency. These problems apply both to the culture of cells and of plant tissues.

Although various attempts have been made to overcome these problems, no method of culturing cells or plant tissues has been developed which satisfactorily deals with the problems.

We have now discovered a process and apparatus which allows cells or plant tissues to be dispersed uniformly in a liquid medium without applying a shear force to the mixture and which, as a result, allows the cells or plant tissues to be cultured highly efficiently.

Accordingly, the present invention consists in a process for culturing cells or plant tissues in which a culture vessel full or at least 90% full of a mixture system comprising a liquid medium containing anchorage-independent cells, plant tissues or anchorage-dependent cells supported on carrier particles is rotated about a horizontal or substantially horizontal axis to rotate the mixture system in the steady state synchronously with the culture vessel and to move said cells, tissues or particles within the mixture system to disperse them in the liquid medium.

The invention also provides apparatus for culturing cells or plant tissues comprising a culture vessel held so that its axis is horizontal or substantially horizontal and a drive mechanism for rotating the culture vessel about a horizontal or substantially horizontal axis, the drive mechanism being capable of rotating in the steady state synchronously with the culture vessel a mixture system comprising a liquid medium with which the culture vessel is filled or substantially filled, said mixture system comprising a liquid medium and anchorage-independent cells, plant tissues or anchorage-dependent cells supported on carrier particles.

The invention is further illustrated by the accompanying drawings, in which:

Figures 1 and 2 are a perspective view and a sectional view, respectively, explaining one embodiment of the invention;

Figure 3 illustrates the motion of particles in the liquid medium;

Figures 4 and 5 are a sectional view and a perspective view, respectively, explaining another embodiment of the invention;

Figure 6 is a sectional view showing one embodiment of apparatus incorporating the apparatus of the present invention;

Figure 7 is a flow chart showing an example of a culture system incorporating the apparatus of the invention;

Figures 8 and 9 are a vertical sectional view and a front view, respectively, illustrating another embodiment of the apparatus of the invention; and

Figure 10 is a sectional view illustrating yet another embodiment of apparatus of the invention.

In the drawings, 1, 12 and 20 represent culture vessels; 2 represents cells or plant tissues; 3 represents a liquid medium; 30 represents a bottom plate; 31 represents a vessel body; 32 represents a depressing mechanism; 35 represents a rotating sleeve; 36 represents dry bearings; 37 represents a stand; 38 represents a jacket, 40 represents a mesh; 41 represents an inner pipe; 42 represents an outer pipe; 50 represents a connecting seal portion; 51 represents a feed pipe; 52 represents a discharge pipe; 60 represents a driven gear; 61 represents a driving gear; 70 represents a liquid medium tank; 71 represents a culture vessel; 72 represents a discharge tank; 73 represents a monitoring mechanism; 74 and 77 represent pH sensors; 75 and 78 represents DO sensors; 80 represents an alkali tank; 81 represents a gas supply pipe; 101 represents a casing; 102 represents a rotating shaft; 103 represents a motor; 104 represents a power transmission mechanism; 105 represents arms; 106 represents culture vessels; 107 represents holders; 201 represents a liquid medium-holding vessel; 202 represents a rotating shaft; 203 represents a medium-circulating pipe; 204 represents a motor; 205 represents a magnet; 206 represents an inner vessel; 207 represents a mesh; 208 represents a medium; 209 represents a pump; and 210 represents a gas supply pipe.

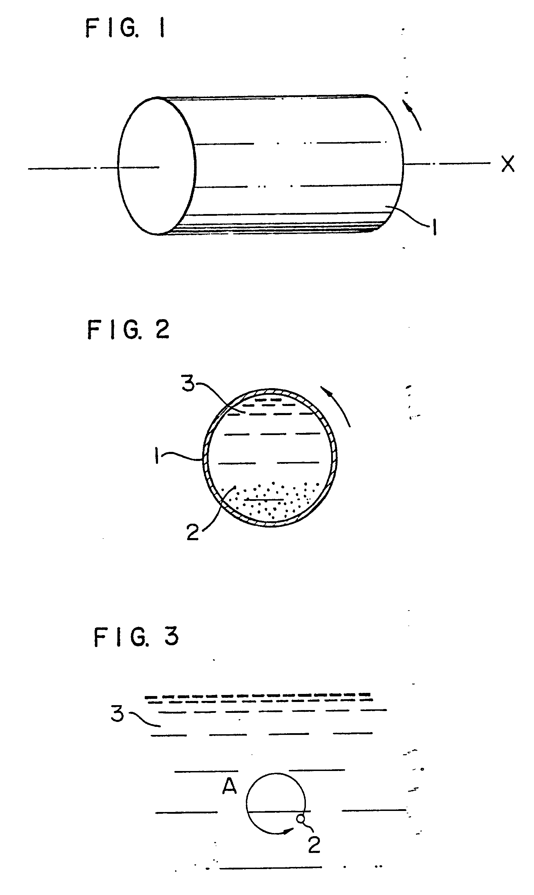

Referring now to Figures 1 and 2, a liquid medium 3 containing cells or plant tissues 2 is packed in, for example, a closed, cylindrical culture vessel 1, so that the interior of the culture vessel 1 is filled or substantially filled with the liquid medium 3. The axis X of the culture vessel 1 is held horizontal or substantially horizontal and the culture vessel 1 is rotated above this axis X at a constant speed. Because of this rotation of the culture vessel 1 and because of the viscosity of the liquid medium 3, when the initial period just after initiation of rotation has elapsed, the mixture system consisting of the culture medium 3 and the cells or plant tissues 2 in the culture vessel 1 rotate about the axis X synchronously with the culture vessel 1, under such conditions that there is no mechanical flow in the culture vessel 1.

In such a system which is rotating synchronously with the culture vessel 1, the cells or plant tissues 2 hardly change their positions relative to each other, because of the viscosity of the liquid medium 3; however, they change their positions relative to the outside of the culture vessel 1 as the vessel rotates. Consequently, the cells or plant tissues 2 are constantly exposed to gravity from gradually varying directions. This can be achieved by appropriate selection of the specific gravity and size of the cells or plant tissues 2, the specific gravity and viscosity of the liquid medium 3, the rotation speed of the culture vessel 1 and other factors.

In such a state, when the cells and plant tissues 2 in the system are observed, each individual cell or tissue, as shown schematically in Figure 3, makes a circular movement, as indicated by arrow A, in the liquid medium 3. It is presumed that this movement causes the cells or plant tissues 2 to be uniformly dispersed in the liquid medium. This dispersion varies depending upon a number of factors but not only occurs very quickly but also occurs uniformly, even in the direction of the axis X. Hence, the cells or plant tissues 2 are very highly dispersed in the liquid medium 3. The dispersion in the direction of the axis X is believed to be because convection currents occur in the liquid medium 3 and these diffuse the cells or plant tissues 2; it is further believed that this is at least partially due to thermodynamic non-uniformity of the distribution state of the cells or plant tissues 2 in the direction of axis X during the period immediately after the start of the rotation.

In accordance with the present invention, it is necessary that the axis about which the culture vessel rotates should be horizontal or substantially horizontal. This is because, if the axis is at a substantial angle to the horizontal, uniform dispersion of the cells or plant tissues in the liquid medium cannot be achieved. The allowable inclination of the axis from the horizontal position will vary depending upon many factors, including the shape of the culture vessel, but, in general, it is preferred that the inclination should be 20° or less. The inclination is more preferably 10° or less, still more preferably 5° or less and most preferably 3° or less.

In any event, the cells or plant tissues should be moved in the liquid medium, without relatively disturbing the liquid medium, and should be uniformly dispersed in the medium; this ensures that the cells or plant tissues are always in contact with fresh liquid medium and that the desired culture can be achieved highly efficiently. The cells or plant tissues are not acted upon by any force accompanied by shear strength or powerful impact and accordingly are not damaged; as a result, even fragile cells or plant tissues which are susceptible to damage can safely be cultured.

No special technique is required in the present invention to fill or substantially fill the culture vessel with the liquid medium and the cells or plant tissues. A prepared mixture of the liquid medium and the cells or plant tissues can simply be placed in the culture vessel. When the cells are anchorage-dependent cells, adhesion of such cells to carrier particles is carried out more effectively in the culture vessel than before they are charged into the culture vessel. Thus, we prefer that the culture vessel should be filled with the cells and carrier particles together with the liquid medium and then the vessel subjected to the rotational procedure of the present invention. This causes the cells and carrier particles to be dispersed uniformly in the liquid medium without their experiencing any shear force and the cells are thus allowed to adhere to the surface of each carrier particle evenly and efficiently. In this case, this adhesion step can be followed by the cultivation procedure of the present invention.

In general, when cultivating cells or plant tissues, it is necessary to replace used liquid medium by fresh medium; such replacement is necessary in the present invention, also, when the cultivation is conducted for a long period of time. Various techniques can be employed for replacing used liquid medium. For example, rotation of the culture vessel may be stopped and then part or all of the liquid medium removed and replaced by fresh medium. Alternatively, the culture vessel may be equipped with a pipe for supplying liquid medium and a pipe for discharging liquid medium and, during cultivation, fresh liquid medium may be fed to the vessel whilst discharging used medium continuously through these pipes. By supplying fresh liquid medium, a cultivation process which usually takes at least a few days can be carried out effectively. If the fresh liquid medium is constantly supplied through a supply pipe, the rate of feed and discharge of liquid medium can be very small; this can prevent or essentially prevent the occurrence of mechanical flow, such as laminar flow or turbulent flow, within the liquid medium, and the dispersion of the cells or plant tissues in the liquid medium can be maintained. Constant supply of fresh liquid medium through a supply pipe is preferable as it increases culture efficiency.

When employing anchorage-dependent cells in the process of the invention, and feeding fresh liquid medium continuously or continually to the culture vessel and withdrawing part of the liquid medium continuously or continually from the culture vessel, if the carrier particles and anchorage-dependent cells are fed to the culture vessel together with the liquid medium, whilst discharging part of the carrier particles on which propagated anchorage-dependent cells have adhered together with the spent liquid medium, it is possible to achieve continuous replacement of liquid medium, adhesion of anchorage-dependent cells to the carrier particles and withdrawal of cultured and propagated cells.

The present invention can be applied to a very wide range of cells and plant tissues. Examples of some such cells and the products thereof are shown in the following Table. In this Table, the cells marked with an asterisk are anchorage-dependent cells, whilst those without an asterisk are anchorage-independent cells.

TABLE

| Physiological active substance to be produced | Cell |
|---|---|
| Interferon | Human normal diploid cell*, Namalba cell, B lymphocyte, T lymphocyte, Fibroblast, Macrophage |
| Interleukin 2 | Jurkat-FHCRC cell, Jurkat cell, JAX cell (e.g. J-111), EL-4 cell, T lymphocyte, FS-6 |
| Interleukin 3 | WEHI-3 cell |
| $\eta$-Interferon | T lymphocyte, MO cell |
| Macrophage activating factor | T lymphocyte |
| B cell growth factor | T lymphocite |
| Human erythropoietin | Human kidney normal cell*, Human kidney tumor cell*, Kidney cancerous cell or its synkaryon* |
| Human growth hormone releasing factor | Cerebrum nuclei arcuati cell* |
| Clony stimulating factor | Human lung cancer-derived cell, KONT strain |
| Monoclonal antibody | Synkaryon between B lymphocyte and human myeloma cell, Synkaryon between lymphocyte and mouse myeloma cell |
| Urokinase | Human kidney normal cell*, Human kidney tumor cell*, Kidney cancerous cell* |
| Tissue plasminogen activator | HeLa cell*, Bowes cell, QG-90 cell*, Hemangioendothelio cell*, Thymus cell*, Thyroid gland cell*, Human kidney normal cell* |

Other anchorage-independent cells to which this invention can be applied, include, for example, lymphoblasts, Burkitts' lymphoma cells, acute lymphoblast-type leukemia cells and myeloma cells such as myeloma and the like. Other anchorage-dependent cells to which this invention can be applied include, for example, human hysterocarcinoma cells HeLa, Chinese hamster lung cells V-79, human foetus lung cells MRC-5, chimpanzee liver fibroblasts, human preputium cells, chicken foetus fibroblasts, first generation monkey kidney cells, metastasized mouse fibroblast cells, pituitary gland tumour cells, green monkey kidney cells Vero, renal gland tumour cells, and the like.

Plant tissues to be cultured in this invention may be chosen freely, and examples include protoplasts, cells, calluses, particular tissues. etc. of plants, such as Goptic japonica Makino forma brachypetale Makino protoplast, Euphorbia Millii protoplast, tobacco mesophyll cell protoplast, carrot protoplast, Marchantia polymorpha callus, soybean callus, Lithospermum erythrorhizon callus, strawberry shoot apex, tomato shoot apex, Solanum goniocalyx shoot apex, pea shoot apex, carnation shoot apex, asparagus shoot apex, chlorella, and the like.

The liquid medium used in this invention for cell-culturing is not critical. Known media which may be used as they are include RPMI 1640 medium, Eagle MEM medium, Dulbecco-modified Eagle medium, Earle medium 199, Ham F12 medium and the like. Preferably, these media are used after adding thereto from 5 to 10% by volume of, for example, bovine foetus serum, newly born cattle serum, equine serum or human serum. Also there can be used serum-free media, for example, HB 102 (HANA BIOLGICS Co.) and RITC 55-9 medium.

The liquid medium used in this invention for culturing plant tissues is likewise not critical. Known media which may be used as they are include Linsmaier-Skoog liquid medium, Murashige-Skoog liquid medium, MG-5, etc.

The precise details of culture media and conditions are not, of course, important to the present invention and will be chosen having regard to the nature of the cells to be cultivated and the products to be produced. As a general guide, however, for cell culture, the liquid medium used in this invention will generally have a specific gravity of from 1.00 to 1.05. It is necessary that oxygen and carbon dioxide should be dissolved in the liquid medim. The liquid medium will normally be maintained at a temperature of from 30 to 40°C, preferably from 36 to 37°C, during cultivation. For the cultivation of plant tissues, the liquid medium will likewise normally have a specific gravity of from 1.00 to 1.05. Oxygen should be dissolved in the liquid medium. The liquid medium will normally have a temperature of from 10 to 40°C, more preferably from 20 to 30°C, during cultivation.

The carrier particles used when culturing anchorage-dependent cells are not critical to the present invention and any carrier particles suitable for adhesion and propagation of anchorage-dependent cells may be employed. Examples of suitable particles include particles whose surfaces are formed by a synthetic polymer (e.g. polystyrene) or a natural polymer (e.g. a protein or polysaccharide). Carrier particles having magnetic properties are preferred, since the collection, transfer and other handling of such particles can be achieved easily and quickly by using a magnet. Magnetic carrier particles can be obtained by binding a powder of magnetic material to a polymer or by coating a magnetic core with a polymer. Examples of magnetic materials include iron, cobalt, nickel, their alloys, low carbon steel, silicon steel, γ-type iron oxide, ferrite and magnetite.

The appropriate specific gravity of the carrier particles will vary depending upon the viscosity and specific gravity of the liquid medium, but it is generally in the range from 1.0 to 1.5. The diameter of the carrier particles is preferably from 40 to 500 μm. The particles are preferably spherical, although they may be granular, cylindrical or amorphous.

The number of cells or plant tissues to be inoculated per ml of the liquid medium is generally from $1 \times 10^4$ to $1 \times 10^6$. The number of carrier particles to be used for culturing anchorage-dependent cells is generally from $1 \times 10^4$ to $1 \times 10^7$ per ml of liquid medium.

Under these conditions, the specific gravity of the cells or plant tissues may be lower than that of the liquid medium. Cells and plant tissues having a specific gravity lower than that of the liquid medium are moved and dispersed uniformly in the liquid medium by buoyancy, rather than by gravity, whilst cells or plant tissues having a specific gravity greater than that of the liquid medium are moved and dispersed by gravity.

In the present invention, it is desirable that the culture vessel should be filled with the mixture system comprising the liquid medium and the cells or plant tissues, so that substantially no space is left inside the culture vessel. In such a state, it is possible to rotate the mixture system synchronously with the culture vessel. Even if some space is left inside the culture vessel, provided the space is very small, any disturbance in the liquid medium due to the presence of the space is limited to a very small portion of the top of the mixture system in the culture vessel and thus the desirable results achievable by the present invention are not significantly impaired. However, if the proportion of space in the culture vessel is large, it is impossible to rotate the mixture system synchronously with the culture vessel without causing disturbances in the mixture system and hence the objects of the present invention cannot be achieved. In the present invention, in most cases, the culture vessel is preferably regarded as "substantially full" when 80% or more of the volume of the culture vessel is filled with the mixture system. More preferably, this amount is 90% by volume or more and most preferably is 98% by volume or more.

The rotational speed of the culture vessel cannot be specified in specific terms, as it will vary depending upon various factors, including the size and specific gravity of the cells or plant tissues, the viscosity of the liquid medium and the shape and size of the culture vessel. However, the speed is usually from 5 to 50 rpm, more preferably from 10 to 30 rpm.

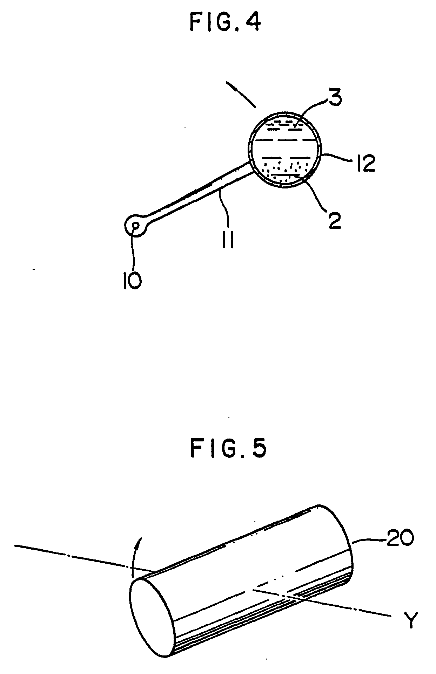

The shape of the culture vessel is preferably cylindrical, as shown in Figure 1, although it may be prismatic or spherical. The method of rotating the culture vessel is not important, provided that the rotation takes place in a substantially perpendicular plane, so that the top and bottom of the culture vessel regularly exchange places. For example, as shown in Figure 4, the culture vessel 12 may be fixed at the distal end of an arm 11, which rotates on a horizontal shaft 10, so as to undergo circular movement in a perpendicular plane. Alternatively, as shown in Figure 5, a cylindrical culture vessel 20 can be rotated about the horizontal axis Y which extends radially of the vessel.

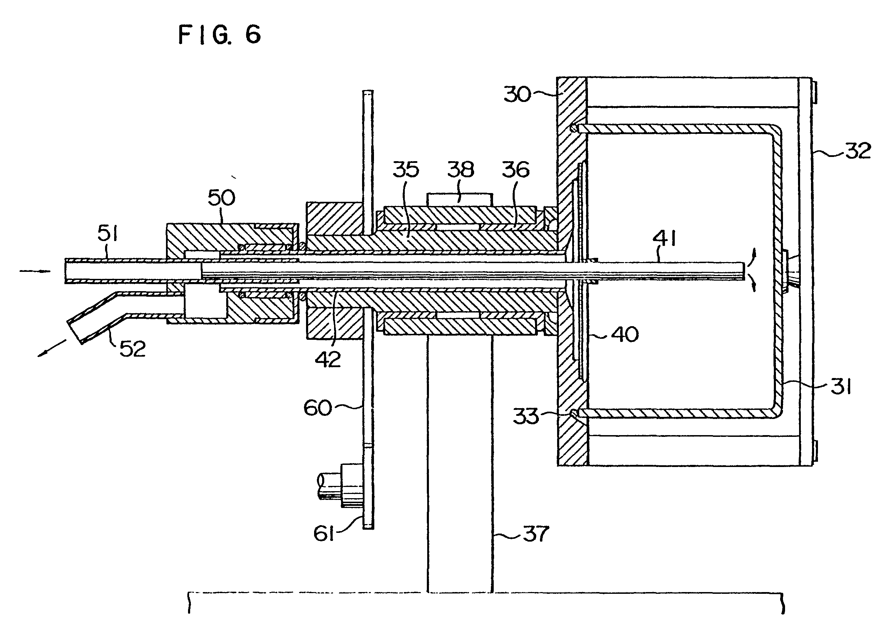

Figure 6 illustrates one embodiment of the present invention. In the apparatus shown in this Figure, the culture vessel consists of a bottom plate 30 and a cylindrical vessel body 31. The vessel body 31 is pressed against the bottom plate 30 by a depressing mechanism 32 fixed to the bottom plate through an O-ring 33. The interior of the culture vessel is used for cultivation. The bottom plate 30 is fixed to a rotating sleeve 35; this rotating sleeve 35 is held horizontally and rotatably through dry bearings 36 by a jacket member 38 supported on a stand 37. The bottom plate 30 has a central opening blocked by a mesh 40, through which liquids can pass but cells or plant tissues cannot pass. An inner pipe 41 passes through this mesh 40, has an opening at its front end and extends through the rotating sleeve 35. The rear end of the inner pipe 41 communicates with a supply pipe 51, so that the inner pipe 41 can rotate freely at the connecting seal portion 50. An outer pipe 41 is fixed to the inner surface of the rotating sleeve 35, so that the outer pipe 42 and inner pipe 41 together form a double pipe. The front end of the outer pipe 42 is fitted to the opening in the bottom plate 30, so as to allow communication between the outer pipe 42 and the interior of the cultivation vessel. The rear end of the outer pipe 42 communicates rotatably with a discharge pipe 52 at the connecting seal portion 50. A driven gear 60 is fixed on the rotating sleeve 35 and engages with a driving gear 61 driven by a motor (not shown).

In the above embodiment, the rotating sleeve 35 is rotated by driving the driving gear 61, which causes the culture vessel, consisting of the bottom plate 30 and the vessel body 31, to be rotated about its horizontal axis; concurrently, the inner pipe 41 and the outer pipe 42 also rotate. Owing to this mechanism, when the culture vessel is substantially filled with liquid medium and cells or plant tissues, the cells or plant tissues are uniformly dispersed in the liquid medium.

In the apparatus shown in Figure 6, feed pipe 51 communicates with the inner pipe 41, and the outer pipe 42 communicates with discharge pipe 52. This mechanism allows fresh liquid medium to be supplied continuously to the culture vessel and spent liquid medium (which has a reduced nutrient content as a result of culturing) to be continuously discharged from the vessel at a flow rate corresponding to the rate of feed of the fresh medium whilst rotating the culture vessel and dispersing the cells or plant tissue and without discharging the cells or plant tissue, which are held back by the mesh 40. This allows the cells or plant tissues to be cultivated over a long period of time.

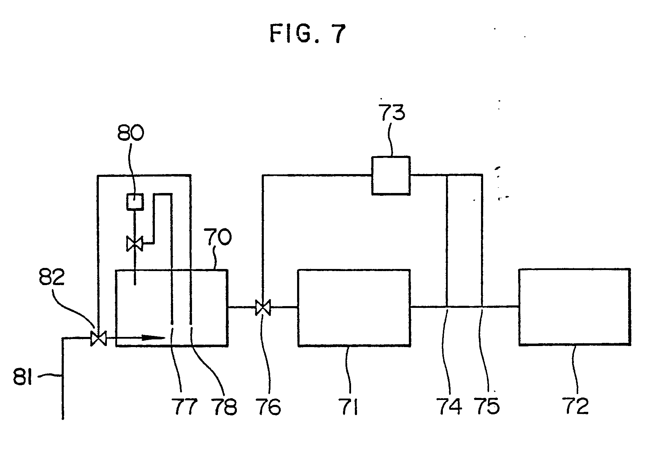

Figure 7 is a flow chart showing one example of a culture system including the apparatus of the present invention. In this system, fresh liquid medium from tank 70 is fed to a culture vessel 71 and spent liquid medium is discharged to discharge tank 72. The discharged medium from culture vessel 71 is monitored by monitoring mechanism 73, to which is fed information from a pH sensor 74 and a DO sensor 75 (which detects the amount of dissolved oxygen). These results may be used to control the amount of fresh medium supplied to the culture vessel 71 by control valve 76. A pH sensor 77 and a DO sensor 78 are provided in the liquid medium tank 70 and alkali is added to this tank from an alkali tank 80 in an amount adjusted in accordance with the pH of the liquid medium as measured by pH sensor 77, so as to control the pH in the liquid tank 70. Air containing, for example, 5% of carbon dioxide is fed to the liquid medium tank 70 through gas supply pipe 81 in an amount adjusted by a control valve 82 in accordance with the dissolved oxygen level detected by DO sensor 78.

When such a culturing system is employed, good culturing conditions can always be maintained; cells or plant tissues can be dispersed uniformly in a liquid medium at a high degree of dispersion; and the desired culture can be conducted highly efficiently on an industrial scale.

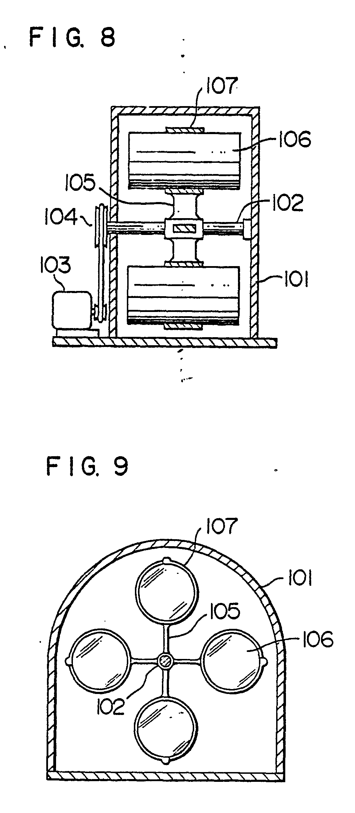

Figures 8 and 9 show another embodiment of apparatus of the present invention. In this embodiment, a rotating shaft 102 extending in the horizontal direction is mounted within a casing 101 provided with a heat-insulating layer. One end of the shaft 102 is connected to a motor 103 (located outside casing 101) through a power transmission mechanism 104. A plurality of arms 105 (4 arms in the embodiment shown in Figures 8 and 9) is fixed to the rotating shaft 102 and the arms project radially from that shaft. At the distal end of each arm 105 is provided a holder 107, which holds a cylindrical culture vessel 106 in a manner which allows the vessel to be attached or detached. Inside the casing 101 is provided a heater (not shown).

When such apparatus is used, in which a plurality of culture vessels 106 are substantially filled with the liquid medium and the cells or plant tissues, the cells or plant tissues can be appropriately and uniformly dispersed in the liquid medium, as explained above, by rotating the rotating shaft 102.

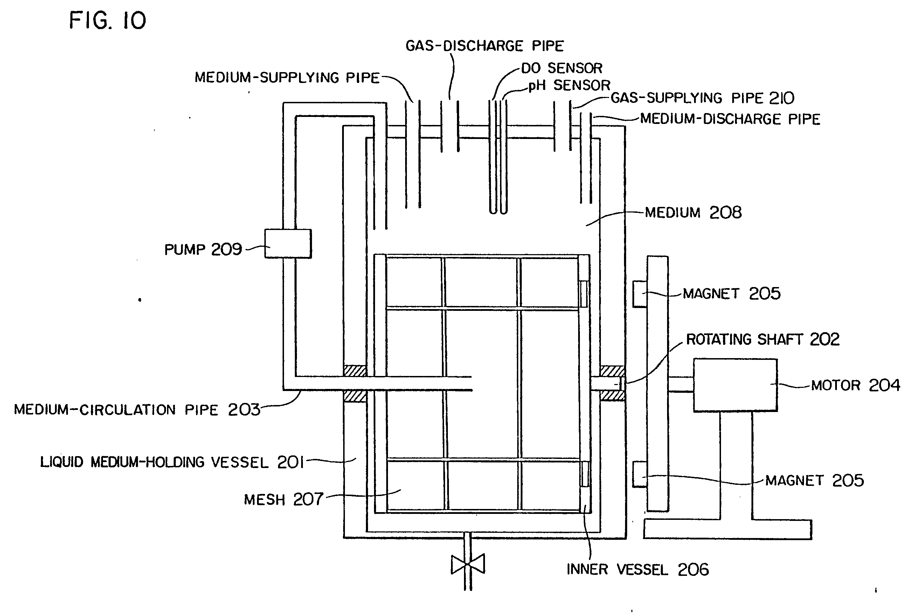

Figure 10 shows a further embodiment of apparatus in accordance with the present invention. In this embodiment, a rotating shaft 202 and a medium-circulating pipe 203 are mounted horizontally in a liquid medium-holding vessel 201. Rotatory power is transmitted to the inner vessel 206 by a magnet 205 located outside the vessel 201. A part or the whole of the side of the inner vessel 206 consists of a porous material (such as mesh 207) through which the cells or plant tissues cannot pass. Medium 208 passes through the inner vessel 206, whilst the cells and plant tissues remain inside the inner vessel. The speed of exchange of the medium between the inside and the outside of the inner vessel 206 can be increased by supplying, if necessary, medium 208 from outside the inner vessel 206 to the inside through the medium-circulation pipe 203 via pump 209. At the top of the vessel 201, for example, air containing 5% of carbon dioxide is supplied to the vessel 201 through a gas-supply pipe 210, which enables the composition of the medium 208 to be maintained in a steady state. If necessary, the air may be supplied directly to the medium 208 by bubbling, which increases the amount of oxygen dissolved in the medium, without air bubbles remaining in the inner vessel 206.

The invention is further illustrated by the following Examples.

Example 1

Cells to be cultured: "V-79" derived from Chinese hamster lung fibroblast

Liquid medium: "Eagle-MEM" containing dissolved oxygen and carbon dioxide, also 10% of bovine foetus serum, (viscosity, 0.01 poise; specific gravity, 1.01)

Carrier particles: "Cytodex III" (Pharmacia Co.); (Particle diameter, 180 μm; specific gravity, 1.03)

In a 300 ml, cylindrical culture vessel were placed 750 mg (dry weight) of carrier particles (about $3 \times 10^6$ particles). The culture vessel was then filled with a liquid medium, and then $2 \times 10^7$ cells to be cultured were inoculated. The culture vessel was tightly sealed to prevent air from entering. The culture vessel was rotated at 15 rpm for 4 hours at a temperature of 37°C.

As a result, the proportion of the carrier particles to which no cells adhered was about 5%; the number of cells adhered to one carrier particle ranged from 2 to 4—the average number was 3.2; and the total number of cells adhered to carrier particles was $1.6 \times 10^7$. Separately, the same culture vessel containing the same substances was rotated for 72 hours under the same conditions, except that, in this case, every 24 hours the rotation was stopped and the whole of the liquid medium was replaced by fresh liquid medium. As a result, the total number of cells propagated on the carrier particles was $3 \times 10^8$.

Example 2

Using the same cells, liquid medium and carrier particles as in Example 1, cell-culture was effected in a culture apparatus having a 1 litre culture vessel as shown in Figure 6. That is, 3 g (dry weight) of carrier particles (about $1.2 \times 10^7$ particles) and 1 litre of a liquid medium were mixed; $1 \times 10^4$ cells to be cultured were inoculated per ml of the liquid medium; the culture vessel was filled with the resulting mixture system so that no air remained inside the culture vessel; and the culture vessel was rotated at 12 rpm for 144 hours while supplying fresh liquid medium to the culture vessel at a rate of 80 ml/hour and discharging the used liquid medium from the culture vessel at the same rate. As a result, the number of cells on the carrier particles was, on average, $3.7 \times 10^6$ per ml of the liquid medium.

Comparative example 1

Using the same cells, liquid medium and carrier particles as in Example 1, cell-culture was effected in a 2 litre spinner bottle. That is, 3 g (dry weight) of carrier particles and 1 litre of a liquid medium were placed in the spinner bottle; then, $1 \times 10^4$ cells to be cultured were inoculated; and culture was effected for 144 hours at temperature of 37°C while rotating the bottle at a rotor speed of 30 rpm. In this case, air containing 5% of carbon dioxide was continuously supplied to top of the spinner bottle to allow the liquid medium to be always in contact with the air. As a result, the number of cells on the carrier particles was $5.1 \times 10^5$, on average, per ml of the liquid medium.

Comparative example 2

As in Example 1, 750 mg (dry weight) of carrier particles (about $3 \times 10^6$ particles) were placed in a 300 ml cylindrical culture vessel, and then the culture vessel was filled with a liquid medium, followed by the inoculation of $2 \times 10^7$ cells to be cultured. The culture vessel was tightly sealed to prevent the air from entering. The rotating shaft of the culture vessel was inclined at an angle of 30°, 45° or 60° to the horizontal, and the culture vessel was rotated at 15 rpm for 4 hours at a temperature of 37°C.

As a result, the total number of cells adhered to the carrier particles was $1.3 \times 10^7$ in the case of the 30° inclination, $1.0 \times 10^7$ in the case of the 45° inclination and $9.5 \times 10^6$ in the case of the 60° inclination. Separately, the same culture vessel containing the same substances was rotated for 72 hours under the same conditions, except that, in this case, every 24 hours the rotation was stopped and the whole of the liquid medium was replaced by fresh liquid medium. As a result, the total number of cells on the carrier particles was $1.9 \times 10^8$ in the case of the 30° inclination, $9.0 \times 10^7$ in the case of the 45° inclination and $8.1 \times 10^7$ in the case of the 60° inclination.

Comparative example 3

As in Example 1, 750 mg (dry weight) of carrier particles (about $3 \times 10^6$ particles) were placed in a 300 ml cylindrical culture vessel, and then the culture vessel was filled with a liquid medium, followed by the inoculation of $2 \times 10^7$ cells to be cultured. The culture vessel was tightly sealed to prevent air from entering. The culture vessel was rotated at 15 rpm for 4 hours at a temperature of 37°C in such a way that the rotational direction was reversed when the vessel was rotated at 180°.

7

As a result, the proportion of carrier particles to which no cells adhered was about 35%, and the total number of cells adhered to carrier particles was $1.2 \times 10^7$. Separately, the same culture vessel containing the same substances was rotated for 72 hours under the same conditions, except that, in this case, every 24 hours the rotation was stopped and the whole of the liquid medium was replaced with fresh liquid medium. As a result, the total number of cells on the carrier particles was $1.3 \times 10^8$.

Comparative example 4

Using the same culture vessel, cells to be cultured, liquid medium and carrier particles as in Example 1, an experiment was conducted to examine the effect of the presence of air in a culture vessel on the propagation of cells. That is, a mixture system consisting of a liquid medium, cells and carrier particles was placed in the culture vessel in an amount corresponding to 70%, 50% or 30% of the capacity of the culture vessel. Specifically, in the culture vessel were placed (1) 210 ml of the liquid medium, 525 mg of carrier particles and $1.4 \times 10^7$ cells to be cultured, (2) 150 ml of the liquid medium, 375 mg of carrier particles and $1 \times 10^7$ cells to be cultured or (3) 90 ml of the liquid medium, 225 mg of carrier particles and $6 \times 10^6$ cells to be cultured. The culture vessel was rotated at 15 rpm for 72 hours at a temperature of 37°C, and every 24 hours the rotation was stopped and the whole of the liquid medium was replaced by fresh liquid medium. As a result, the total number of cells on the carrier particles was $1.4 \times 10^8$ in the case of the 70% fill, $7 \times 10^7$ in the case of the 50% fill and $5.4 \times 10^7$ in the case of the 30% fill.

Example 3

A 100 ml cylindrical culture vessel was filled with a Linsmaier-Skoog liquid medium having a specific gravity of 1.0. To this was added 0.5 g (dry weight) of the wet callus of *Lithospermum erythrorhizon* and the culture vessel was tightly sealed. Then, the culture vessel was rotated at 15 rpm for 14 days at 25°C to culture the callus.

The plant tissues thus obtained were dried and weighed: they weighed 1.6 g.

Example 4

A 100 ml cylindrical culture vessel was filled with a Linsmaier-Skoog liquid medium. To this was added 0.1 g (dry weight) of tobacco mesophyll protoplast. Culture was conducted for 60 hours at 25°C, while rotating the culture vessel at 15 rpm, supplying fresh liquid medium to the culture vessel at the rate of 120 ml per 24 hours and a discharging the used liquid medium from the culture vessel at the same rate.

The plant tissues thus obtained were dried and weighed: they weighed 1.2 g.

Comparative example 5

Using a 200 ml spinner bottle, 100 ml of the same liquid medium as in Example 3 and 0.5 g of the same callus as in Example 3, culture was conducted in the same manner as in Example 3 while rotating a rotary blade at 15 rpm. The dry weight of the plant tissues thus obtained was 1.0 g.

Comparative example 6

Using the same apparatus and culture conditions as in Example 3, except that the rotating shaft was inclined at 60° to the horizontal, culture was conducted. The plant tissues were not uniformly dispersed in the medium and no propagation was seen.

Example 5

Using a culture vessel having the construction shown in Figure 10 provided with a 1.5 litre liquid medium-holding vessel 201 and a 800 ml inner vessel 206 and using the same cells to be cultured, liquid medium and carrier particles as in Example 1, the cells were cultured. That is, 2.4 g (dry weight) of the carrier particles (about $9.6 \times 10^6$ particles) were placed in the inner vessel 206 in the liquid medium-holding vessel 201 containing 1.2 litres of the liquid medium, and subsequently the cells to be cultured were inoculated in the inner vessel 206 in an amount of $1 \times 10^4$ cells per ml of the liquid medium contained in the inner vessel 206, after which the inner vessel 206 was rotated at 15 rpm at a temperature of 37°C over 144 hours while supplying the liquid medium at the rate of 35 ml/hour to the liquid medium-holding vessel and discharging the used liquid medium at the same rate. In this case, air containing 5% of carbon dioxide was continuously supplied to the upper part of the liquid medium-holding vessel 201, and, when the amount of oxygen dissolved in the liquid medium became less than 2 ppm, the oxygen content in the air was increased to control the amount of oxygen dissolved in the liquid medium to not less than 2 ppm. As a result, the number of cells on the carrier particles was $4.5 \times 10^6$, on average, per ml of the liquid medium in the inner vessel 206.

Reference example

Black particles having a specific gravity of 1.03 and a particle diameter of 180 μm were placed into a 100 ml cylindrical vessel. The vessel was filled with water and then tightly sealed to prevent the entrance of air. The cylindrical vessel was rotated at 15 rpm whilst keeping the rotating shaft at an angle of 0°, 30°, 45° or 60° to the horizontal. It was found by observation with the naked eye that the dispersion of black particles in the

EP 0 164 888 B1

liquid medium was unsatisfactory when the inclination of the rotating shaft of the vessel was greater than 30°.

Specifically, at an inclination of 0°, the particles were observed to be evenly distributed throughout the vessel. At an inclination of 30°, the distribution of particles was still almost uniform throughout the vessel. However, at an inclination of 45°, a pronounced distribution gradient was observed, with virtually no particles present at the "top" of the vessel and a substantial concentration in a thin layer towards the "bottom" of the vessel and relatively few particles inbetween, increasing in concentration towards the "bottom" of the vessel. At an inclination of 60°, almost all of the particles were concentrated in a thin layer towards the "bottom" of the vessel and, although a few particles wsre distributed throughout the remainder of the vessel, the number of these was insignificant. The terms "top" and "bottom" refer to the respective ends of the cylinder.

**Claims**

1. A process for culturing cells or plant tissues in which a culture vessel at least 90% full of a mixture system comprising a liquid medium containing anchorage-independent cells, plant tissues or anchorage-dependent cells supported on carrier particles, and being the whole accumulation of liquid and solid particles distributed therein, is rotated about a horizontal or substantially horizontal axis so as to rotate the mixture system in the steady state synchronously as a unit with the culture vessel but permit said cells, tissues or particles to move within the essentially synchronised mixture system enough to maintain dispersion in and contact with the liquid medium thereof.

2. A process according to Claim 1, in which said culture vessel is rotated about an axis inclined at an angle of no more than 20° to the horizontal.

3. A process according to Claim 2, in which said angle is 10° or less to the horizontal.

4. A process according to any one of the preceding Claims, in which said culture vessel is rotated at a rate of from 5 to 50 rpm.

5. A process according to any one of the preceding Claims, in which the mixture system comprises the liquid medium and carrier particles having adhered thereto anchorage-dependent cells.

6. A process according to Claim 5, in which said carrier particles are magnetic.

7. A process according to Claim 5, or Claim 6, in which said carrier particles have a specific gravity of from 1.0 to 1.5.

8. A process according to any one of Claims 5 to 7 which said carrier particles have a particle diameter of from 40 to 500 µm.

9. A process according to any one of the preceding Claims, in which said liquid medium is continuously supplied to and discharged from said culture vessel.

10. A process according to any one of the preceding Claims, in which said culture vessel is cylindrical and has a rotating shaft which passes through the centre of each end of the cylinder.

11. A process according to Claim 10, in which a part or the whole of a side of the culture vessel consists of a porous material through which the liquid medium can pass but the said cells, tissues or particles cannot pass, and the whole of the culture vessel is immersed in the liquid medium.

12. Apparatus for culturing cells or plant tissues comprising a cylindrical culture vessel and a drive mechanism for rotating the culture vessel about the horizontal or substantially horizontal axis defined by a rotating shaft which passes through the centre of each end of the cylinder, the drive mechanism being capable of rotating in the steady state synchronously and as a unit with the culture vessel a mixture system comprising a liquid medium and anchorage-independent cells, plant tissues or anchorage-dependent cells supported on carrier particles.

13. Apparatus according to Claim 12 in which a part or the whole of a side of the culture vessel consists of a porous material through which said liquid medium can pass but said cells, tissues and particles cannot pass, and the apparatus additionally comprises a liquid medium-holding vessel within which the whole of the culture vessel is placed.

14. Apparatus according to either of Claims 12 or 13 in which said culture vessel has a liquid medium inlet and a liquid medium outlet.

**Patentansprüche**

1. Verfahren zur Züchtung von Zellen oder Pflanzengeweben, bei dem ein Kulturbehälter, der wenigstens 90% voll von einem Mischungssystem ist, das ein verankerungsunabhängige Zellen, Pflanzengewebe oder auf Trägerteilchen gehaltene verankerungsabhängige Zellen enthaltendes flüssiges Medium aufweist, wobei die ganze Ansammlung von flüssigen und festen Teilchen darin verteilt ist, um eine horizontale oder im wesentlichen horizontale Achse derart gedreht wird, daß das Mischungssystem im stetigen Zustand synchron als Einheit mit dem Kulturbehälter rotiert, jedoch ermöglich wird, daß sich die Zellen, Gewebe oder Teilchen innerhalb des im wesentlichen synchronisierten Mischungssystems genügend bewegen, um eine Dispesion in und einen Kontakt mit dessen flüssigem Medium aufrechtzuerhalten.

9

2. Verfahren nach Anspruch 1, bei dem der Kulturbehälter um eine in einem Winkel von nicht mehr als 20° zur Horizontalen geneigte Aschse gedreht wird.

3. Verfahren nach Anspruch 2, bei dem der Winkel 10° oder weniger zur Horizontalen ist.

4. Verfahren nach irgendeinem der vorstehenden Ansprüche, bei dem der Kulturbehälter mit einer Drehzahl von 5 bis 50 U/min gedreht wird.

5. Verfahren nach irgendeinem der vorstehenden Ansprüche, bei dem das Mischungssystem das flüssige Medium und Trägerteilchen mit daran haftenden verankerungsabhängigen Zellen aufweist.

6. Verfahren nach Anspruch 5, bei dem die Trägerteilchen magnetisch sind.

7. Verfahren nach Anspruch 5 oder Anspruch 6, bei dem die Trägerteilchen ein spezifisches Gewicht von 1,0 bis 1,5 haben.

8. Verfahren nach irgendeinem der Ansprüche 5 bis 7, bei dem die Trägerteilchen einen Teilchendurchmesser von 40 bis 500 µm haben.

9. Verfahren nach irgendeinem der vorstehenden Ansprüche, bei dem das flüssige Medium dem Kulturbehälter kontinuierlich zugeführt und von diesem abgeführt wird.

10. Verfahren nach irgendeinem der vorstehenden Ansprüche, bei dem der Kulturbehälter zylindrisch ist und eine Drehwelle hat, die durch die Mittel jedes Endes des Zylinders verläuft.

11. Verfahren nach Anspruch 10, bei dem ein Teil oder die Gesamtheit einer Seite des Kulturbehälters aus einem porösen Material besteht, durch das das flüssige Medium durchtreten kann, jedoch die Zellen, Gewebe oder Teilchen nicht durchtreten können, und die Gesamtheit des Kulturbehälters in das flüssige Medium eingetaucht wird.

12. Anlage zur Züchtung von Zellen oder Pflanzengeweben mit einem zylindrischen Kulturbehälter und einem Antriebsmechanismus zum Drehen des Kulturbehälters um die horizontale oder im wesentlichen horizontale Achse, die durch eine Drehwelle gebildet wird, die durch die Mittel jedes Endes des Zylinders verläuft, wobei der Antriebsmechanismus dazu geeignet ist, im stetigen Zustand synchron und als eine Einheit mit dem Kulturbehälter ein Mischungssystem rotieren zu lassen, das ein flüssiges Medium und verankerungsunabhängige Zellen, Pflanzengewebe oder auf Trägerteilchen gehaltene verankerungsabhängige Zellen aufweist.

13. Anlage nach Anspruch 12, bei der ein Teil oder die Gesamtheit einer Seite des Kulturbehälters aus einem porösen Material besteht, durch das das flüssige Medium durchtreten kann, jedoch die Zellen, Gewebe und Teilchen nicht durchtreten könen, und die Anlage zusätzlich ein ein flüssiges Medium enthaltendes Gefäß aufweist, innerhalb dessen die Gesamtheit des Kulturbehälters angeordnet ist.

14. Anlage nach entweder Anspruch 12 oder Anspruch 13, bei der der Kulturbehälter einen Flüssigmediumeinlaß und einen Flüssigmediumsauslaß hat.

## Revendications

1. Procédé pour cultiver des cellules ou des tissus végétaux, dans lequel un récipient de culture rempli à 90% au moins d'un système en mélange comprenant un milieu liquide contenant des cellules indépendantes d'un ancrage, des tissus végétaux ou des cellules dépendantes d'un ancrage supportées sur des particules de support, et consistant en la masse totale du liquide et des particules solides qui y sont réparties, est mis en rotation autour d'un axe horizontal ou sensiblement horizontal de façon à faire tourner le système en mélange en régime permanent, de façon synchrone, comme un tout avec le récipient de culture, mais à permettre auxdites cellules ou particules ou auxdits tissus de se déplacer suffisamment au sein du système en mélange essentiellement synchronisé pour se maintenir en dispersion dans, et en contact avec, le milieu liquide fe celui-ci.

2. Procédé selon la revendication 1, dans lequel ledit récipient de culture est mis en rotation autour d'un axe incliné d'un angle non supérieur à 20° par rapport à l'horizontale.

3. Procédé selon la revendication 2, dans lequel ledit angle est de 10° ou moins par rapport à l'horizontale.

4. Procédé selon l'une quelconque des revendications précédentes, dans lequel ledit récipient de culture est mis en rotation à une vitesse de 5 à 50 tr/min.

5. Procédé selon l'une quelconque des revendications précédentes, dans lequel le système en mélange comprend le milieu liquide et les particules de support sur lesquelles adhèrent des cellules dépendantes d'un ancrage.

6. Procédé selon la revendication 5, dans lequel lesdites particules de support sont magnétiques.

7. Procédé selon la revendication 5 ou la revendication 8, dans lequel lesdites particules de support ont une densité de 1,0 à 1,5.

8. Procédé selon l'une quelconque des revendications 5 à 7, dans lequel lesdites particules de support ont un diamètre particulaire de 40 à 500 µm.

9. Procédé selon l'une quelconque des revendications précédentes, dans lequel ledit milieu liquide est continuellement envoyé audit récipient de culture de évacué de celui-ci.

10. Procédé selon l'une quelconque des revendications précédentes, dans lequel ledit récipient de culture est cylindrique et comporte un arbre rotatif qui passe par le centre de chaque extrémité du cylindre.

11. Procédé selon la revendication 10, dans lequel une partie ou la totalité d'un côté du récipient de culture est constituée d'un matériau poreux que le milieu liquide peut traverser mais que lesdites cellules

# EP 0 164 888 B1

ou particules ou lesdits tissus ne peuvent pas traverser, et la totalité du récipient de culture est immergée dans le milieu liquide.

12. Appareil pour cultiver des cellules ou des tissus végétaux, comprenant un récipient de culture cylindrique et un mécanisme moteur destiné à mettre en rotation le récipient de culture autour de l'axe horizontal ou sensiblement horizontal défini par un arbre rotatif moteur étant capable de faire tourner en régime permanent de façon synchrone et comme un tout avec le récipient de culture un système de mélange comprenant un milieu liquide et des cellules indépendantes d'un ancrage, des tissus végétaux ou des cellules dépendantes d'un ancrage supportées sur des particules de support.

13. Appareil selon la revendication 12, dans lequel une partie ou la totalité d'un côté du récipient de culture est constituée d'un matériau poreux que peut traverser ledit milieu liquide mais que lesdites cellules ou particules ou lesdits tissus ne peuvent pas traverser, et l'appareil comprend de plus un récipient contenant du milieu liquide dans lequel est placée la totalité du récipient de culture.

14. Appareil selon l'une ou l'autre des revendications 12 ou 13, dans lequel ledit récipient de culture comporte une entrée de milieu liquide et une sortie de milieu liquide.

F I G. 1

X

1

F I G. 2

3

1

2

F I G. 3

3

A

2

1

# FIG. 4

# FIG. 5

FIG. 6

FIG. 7

FIG. 8

FIG. 9

## FIG. 10

GAS-DISCHARGE PIPE

DO SENSOR

pH SENSOR

MEDIUM-SUPPLYING PIPE

GAS-SUPPLYING PIPE 210

MEDIUM-DISCHARGE PIPE

MEDIUM 208

PUMP 209

MAGNET 205

ROTATING SHAFT 202

MOTOR 204

MEDIUM-CIRCULATION PIPE 203

LIQUID MEDIUM-HOLDING VESSEL 201

MAGNET 205

MESH 207

INNER VESSEL 206